# EUROPÄISCHE PATENTSCHRIFT

(11) **EP 1 110 565 B1**
(45) Veröffentlichungstag und Bekanntmachung des Hinweises auf die Patenterteilung: **23.07.2008**
(21) Anmeldenummer: 00127880.3
(22) Anmeldetag: 20.12.2000
(51) Int. Cl.: A61M 1/28

(54) **Vorrichtung zur Peritonealdialyse**
Peritoneal dialysis apparatus
Appareil de dialyse péritonéale

(30) Priorität: 23.12.1999 DE 19962315
(43) Veröffentlichungstag der Anmeldung: 27.06.2001
(73) Patentinhaber: Fresenius Medical Care Deutschland GmbH, 61352 Bad Homburg v.d.H. (DE)
(72) Erfinder: Noack, Joachim, Dr., 97616 Neustadt (DE)
(74) Vertreter: Luderschmidt, Schüler & Partner

(56) Entgegenhaltungen:
- EP-A- 0 499 718
- DE-A- 3 113 934
- DE-A- 4 443 714
- US-A- 4 338 190
- US-A- 5 498 338

## Beschreibung

Die Erfindung betrifft eine Vorrichtung zur Peritonealdialyse zum Anschluß an einen implantierbaren Peritonealkatheter und eine Vorrichtung zur Peritonealdialyse mit einem implantierbaren Peritonealkatheter, wobei die Vorrichtung zur Peritonealdialyse, ein Überleitungsschlauchsystem und eine Einrichtung zur Bereitstellung einer Peritoneallösung aufweist, und der Peritonealkatheter ein erstes Lumen zum Zuführen der Peritoneallösung und ein zweites Lumen zum Abführen der Peritoneallösung aus dem Peritoneum des Patienten aufweist.

Bei der kontinuierlichen ambulanten Peritonealdialyse (CAPD) wird über einen permanent implantierten Peritonealkatheter und ein Überleitungsschlauchsystem aus einem Plastikbeutel das Dialysat unter dem Einfluß der Schwerkraft in den Peritonealraum, d.h. die Bauchhöhle des Patienten instilliert. Das Dialysat verbleibt mehrere Stunden in der Bauchhöhle. Nach Ablauf dieses Zyklus wird das Dialysat wieder über das Schlauchsystem abgeführt. Durch den Austausch von Flüssigkeit und gelösten Stoffen zwischen dem Blut in den peritonealen Kapillaren und dem Dialysat kann eine ausreichende Elimination harnpflichtiger Substanzen erfolgen.

Bei der Peritonealdialyse muß die dem Peritonealraum zugeführte Peritoneallösung absolut steril sein, da es ansonsten zu der gefürchteten Peritonitis kommen kann.

Aus der EP-A-0 149 001 ist ein Peritonealdialysegerät bekannt, das über einen Dialysator verfügt, der durch eine Membran in eine erste und zweite Kammer geteilt ist. Die erste Kammer ist über einen ersten sterilen Kreislauf mit dem Peritonealkatheter verbunden, während die zweite Kammer über einen nicht-sterilen Kreislauf mit einer Vorrichtung zur Bereitstellung von Dialysierflüssigkeit verbunden ist. Die Kreisläufe sind durch die Membran des Dialysators voneinander getrennt. An der Membran erfolgt die Abscheidung der in der Peritoneallösung enthaltenden Stoffwechselprodukte infolge Diffusion durch die Membran in die nicht-sterile Dialysierflüssigkeit. Ein Peritonealdialysegerät mit zwei Membranfiltern ist aus der US-4,338,190 bekannt.

Der Peritonealkatheter weist zwei Lumen auf, wobei die Peritoneallösung über das erste Lumen dem Peritoneum zugeführt und über das zweite Lumen wieder abgeführt wird. Nachteilig ist, daß aufgrund des relativ geringen Querschnitts der Lumen hierfür verhältnismäßig viel Zeit erforderlich ist.

Aus der US-A-5 498 338 ist eine Vorrichtung zur Peritonealdialyse bekannt, die über einen Peritonealkatheter verfügt, der zwei Lumen aufweist. Zu dem ersten Lumen des Peritonealkatheters führt eine Zulaufleitung, während von dem zweiten Lumen des Katheters eine Ablaufleitung abgeht. In der Zulaufleitung ist eine erste Pumpe und in der Ablaufleitung eine zweite Pumpe angeordnet. Für den Einlauf von Peritoneallösung in den Peritonealraum wird die Pumpe in der Zulaufleitung und für den Auslauf der Peritoneallösung aus dem Peritonealraum wird die Pumpe in der Ablaufleitung betrieben. Den Auslass der Pumpe in der Zulaufleitung und den Auslass der Pumpe in der Ablaufleitung verbindet eine Verbindungsleitung, in der ein erstes Sperrorgan angeordnet ist. Ein zweites Sperrorgan ist in der Zulaufleitung zwischen dem Verbindungspunkt der Verbindungsleitung und dem Katheter angeordnet. Die Verbindungsleitung mit dem ersten Sperrorgan und das zweite Sperrorgan dienen zur Kalibrierung der Pumpen. Die Sperrorgane sind derart geschaltet, dass das Sperrorgan in der Verbindungsleitung geöffnet ist, während das Sperrorgan in der Zulaufleitung geschlossen ist. Alternativ sind beide Sperrorgane geschlossen.

Der Erfindung liegt die Aufgabe zugrunde, eine Vorrichtung zur Peritonealdialyse zu schaffen, bei der Ein- und Auslauf der Peritoneallösung beschleunigt ist.

Die Lösung dieser Aufgabe erfolgt erfindungsgemäß mit den im Patentanspruch 1 angegebenen Merkmalen.

Bei der erfindungsgemäßen Vorrichtung zur Peritonealdialyse ist eine Verbindung mit einem Sperrorgan vorgesehen, mit dem eine Strömungsverbindung zwischen dem ersten und zweiten Lumen des Peritonealkatheters hergestellt werden kann. Zum Befüllen des Peritonealraumes wird das Sperrorgan geöffnet, so daß die Peritoneallösung sowohl über das erste als auch das zweite Lumen des Peritonealkatheters zufließen kann. Während der Dialyse ist das Sperrorgan hingegen geschlossen, um die Peritoneallösung aus dem Peritonealraum über das erste Lumen und die Zulaufleitung in den Dialysator leiten und über die Ablaufleitung und das zweite Lumen wieder dem Peritonealraum zuführen zu können. Der Auslauf der Peritoneallösung erfolgt wieder bei geöffnetem Sperrorgan, wobei die Peritoneallösung sowohl über das erste als auch das zweite Lumen des Peritonealkatheters abläuft. Da jeweils die Querschnitte von zwei Katheterlumen zur Verfügung stehen, sind die Füllung und der Auslauf beschleunigt.

Unter Verbindungsleitung ist jede Verbindung zwischen dem ersten und zweiten Lumen des Peritonealkatheters zu verstehen. Die Verbindungsleitung kann eine die Zulauf- und Ablaufleitung des Überleitungsschlauchsystems verbindende Leitung oder auch ein Kurzschlußstück sein, das eine Verbindung zwischen den beiden Lumen des Peritonealkatheters herstellt. Unter einem Peritonealkatheter wird sowohl ein Katheter verstanden, der aus einem mehrlumigen Schlauch besteht, als auch ein Katheter der mehrere einlumige Schläuche umfaßt.

In einer bevorzugten Ausführungsform der Vorrichtung zur Peritonealdialyse sind zur Steuerung des Flüssigkeitsflusses in der Zulaufleitung ein zweites Sperrorgan und/oder in der Ablaufleitung ein drittes Sperrorgan vorgesehen. Durch Schließen und Öffnen der Sperrorgane kann das erste und zweite Lumen für den Zulauf bzw. Auslauf der Peritoneallösung freigegeben werden.

Die Zulauf- und Ablaufleitung des Überleitungsschlauchsystems können einstückiger Bestandteil des Peritonealkatheters sein. Vorteilhafterweise ist das Überleitungsschlauchsystem aber an dem Peritonealkatheter mittels eines Konnektors angeschlossen, so daß das Schlauchsystem von dem Peritonealkatheter abgenommen werden kann.

Eine besonders bevorzugte Ausführungsform der Peritonealdialysevorrichtung verfügt über einen durch eine Membran in einer erste und zweite Kammer unterteilten Dialysator, wobei die Zulaufleitung mit dem Einlaß der ersten Kammer und die Ablaufleitung des Überleitungsschlauchsystems mit dem Auslaß der ersten Kammer des Dialysators verbunden ist. Der Einlaß der zweiten Kammer ist mit einer Einrichtung zur Bereitstellung von Dialysierflüssigkeit und der Auslaß der zweiten Kammer des Dialysators mit einem Ablauf verbunden. Während die erste Kammer von der Peritoneallösung durchströmt wird, fließt die Dialysierflüssigkeit durch die zweite Kammer des Dialysators, so daß an dessen Membran die Abscheidung der in der Peritoneallösung enthaltenden Stoffwechselprodukte erfolgen kann.

Die Förderung der Peritoneallösung erfolgt vorteilhafterweise durch eine Pumpe, die in die Zulaufleitung geschaltet ist. Die Einrichtung zur Bereitstellung der Peritoneallösung ist vorteilhafterweise ein Behältnis, insbesondere Beutel mit einem Ein-/Auslaß, der über eine Anschlußleitung mit der Zulaufleitung des Überleitungsschlauchsystems verbunden ist. In der Anschlußleitung ist vorteilhafterweise ein viertes Sperrorgan vorgesehen.

Der Verbindungspunkt, an dem die Anschlußleitung der Einrichtung zur Bereitstellung der Peritoneallösung mit der Zulaufleitung verbunden ist, befindet sich vorzugsweise stromauf der Pumpe, die vorzugsweise eine okkludierende Pumpe ist. Während des Auslaufs der Peritoneallösung verhindert die okkludierende Pumpe somit einen Rückfluß der Lösung in den Dialysator.

Die Sperrorgane sind vorzugsweise elektromagnetisch oder pneumatisch betätigbare Schlauchklemmen. Es können aber auch handbetätigte Schlauchklemmen vorgesehen sein.

Im Fall von elektromagnetisch oder pneumatisch betätigbaren Schlauchklemmen ist eine Steuereinheit vorgesehen, mit der die Sperrorgane derart angesteuert werden, daß die einzelnen Phasen, nämlich Füllen, Dialyse und Auslauf automatisch vorgegeben werden.

Im folgenden wird ein Ausführungsbeispiel der Erfindung unter Bezugnahme auf die Zeichnungen näher erläutert.

Es zeigen:
- Figur 1: eine Vorrichtung zur Peritonealdialyse in vereinfachter schematischer Darstellung,
- Figur 2: ein weiteres Ausführungsbeispiel der Vorrichtung zur Peritonealdialyse, die anstelle von Schlauchklemmen über einen Drehschieber verfügt,
- Figuren 3a bis 3c: die unterschiedlichen Stellungen des Drehschiebers der Peritonealdialysevorrichtung von Figur 2 und
- Figuren 4a und 4b: einen Schnitt entlang der Linien A-A und B-B von Figur 3a.

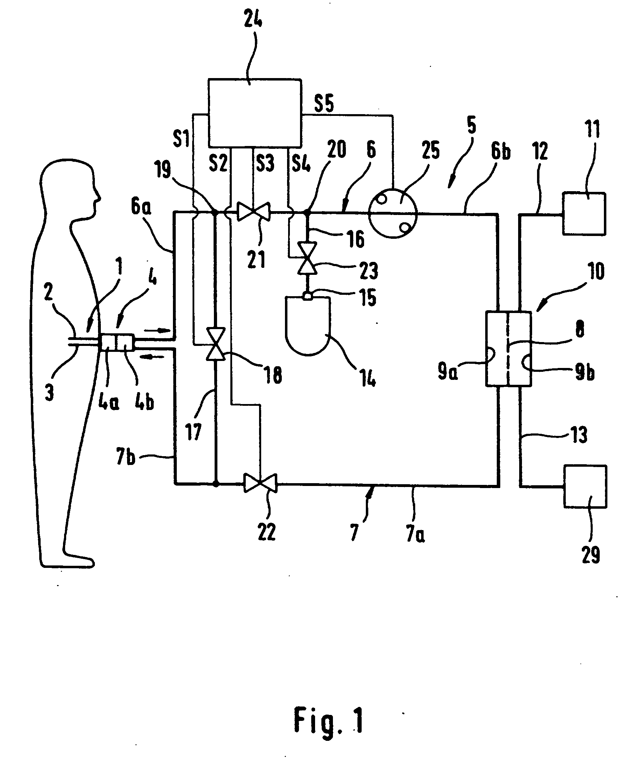

Figur 1 zeigt die wesentlichen Komponenten der Vorrichtung zur Peritonealdialyse in vereinfachter schematischer Darstellung. Die Peritonealdialysevorrichtung umfaßt einen permanent implantierbaren Peritonealkatheter 1, der ein erstes und zweites Lumen 2,3 zum Zuführen einer Peritoneallösung in den Peritonealraum bzw. Abführen der Lösung aus dem Peritonealraum des Patienten aufweist. Derartige zweilumige Peritonealkatheter sind allgemein bekannt, so daß es keiner weiteren Erläuterung bedarf.

Der Peritonealkatheter 1 ist mittels eines Konnektors 4 mit einem Überleitungsschlauchsystem 5 verbunden, das eine Zulaufleitung 6 und eine Ablaufleitung 7 aufweist. Das eine Ende der Zulaufleitung 6 ist mit dem ersten Lumen 2 des Peritonealkatheters verbunden, während das andere Ende der Zulaufleitung mit dem Einlaß einer ersten Kammer 9a eines durch eine semipermeable Membran 8 in die erste Kammer 9a und eine zweite Kammer 9b unterteilten Dialysators 10 verbunden ist. Der Auslaß der ersten Kammer 9a des Dialysators 10 ist mit dem einen Ende der Ablaufleitung 7 verbunden, während das andere Ende der Ablaufleitung mit dem zweiten Lumen 3 des Peritonealkatheters 1 verbunden ist.

Der Dialysator 10 kann Bestandteil einer herkömmlichen Dialysevorrichtung sein. Die Dialysevorrichtung verfügt neben anderen Komponenten, die der besseren Übersicht halber hier nicht dargestellt sind, über eine Einrichtung 11 zur Bereitstellung der Dialysierflüssigkeit, die über eine Zuführleitung 12 mit dem Einlaß der zweiten Kammer 9b des Dialysators 10 verbunden ist. Der Auslaß der zweiten Kammer 9b des Dialysators 10 ist über eine Ablaufleitung 13 mit einem Auslauf 29 verbunden. Während des Betriebs strömt die frische Dialysierflüssigkeit durch die zweite Kammer 9b des Dialysators 10 und wird anschließend verworfen. Dies kann bedeuten, daß die Lösung entweder entsorgt oder wieder aufbereitet wird.

Der Konnektor 4, der aus zwei zusammensteck- bzw. schraubbaren Teilstücken 4a, 4b besteht, erlaubt es, das Überleitungsschlauchsystem von dem permanent implantierten Peritonealkatheter abzunehmen, so daß sich der Patient frei bewegen kann. Andererseits kann jedoch auch das Überleitungssystem mit dem Peritonealkatheter verschweißt sein. Die reversible Trennung der Leitungssysteme erfolgt dann mittels einer speziellen Schlauchschweißmaschine, die zum Stand der Technik gehört.

Die Peritoneallösung, bei der es sich auch um eine Dialysierflüssigkeit handelt, wird in einer Einrichtung 14, vorteilhafterweise einem Beutel bereitgestellt. Der Beutel 14 weist einen Ein-/Auslaß 15 auf, der über eine Anschlussleitung 16 mit der Zulaufleitung 6 verbunden ist. Von der Zulaufleitung 6 zweigt eine Verbindungsleitung 17 ab, die zu der Ablaufleitung 7 führt. In der Verbindungsleitung 17 ist ein erstes Sperrorgan 18 vorgesehen, während in der Zulaufleitung 6 zwischen den Verbindungspunkten 19, 20 der Verbindungs- bzw. Anschlußleitung 17, 16 ein zweites Sperrorgan 21 und in der Ablaufleitung 7 stromauf der Verbindungsleitung ein drittes Sperrorgan 22 vorgesehen sind. In der Anschlußleitung 16 ist ein viertes Sperrorgan 23 vorgesehen. Bei den Sperrorganen handelt es sich um elektromagnetisch betätigbare Schlauchklemmen, die über Steuerleitungen S 1 bis S4 mit einer zentralen Steuereinheit 24 verbunden sind. Anstelle von elektromagnetisch betätigbaren Schlauchklemmen können auch pneumatische Klemmen vorhanden sein.

Die Förderung der Flüssigkeit im Peritoneallösungskreislauf erfolgt mit einer okkludierenden Schlauchpumpe 25, die über eine weitere Steuerleitung S5 von der Steuereinheit 24 angesteuert wird. Die Peritonealdialysevorrichtung kann noch über weitere Komponenten, beispielsweise zusätzliche Pumpen zum Fördern der Flüssigkeiten, Flüssigkeitsspeicher oder Tropfkammern sowie Druckaufnehmer verfügen, die aber der besseren Übersichtlichkeit halber nicht dargestellt sind. Diese Komponenten sind beispielsweise in der EP 0 149 001 beschrieben, die eine bekannte Peritonealdialysevorrichtung betrifft.

Die zentrale Steuerung der Peritonealdialysevorrichtung übernimmt die Steuereinheit 24, von der die Sperrorgane und die okkludierende Pumpe angesteuert werden. Der Arbeitsablauf ist wie folgt.

Zur Einleitung der Peritonealdialyse wird in einer ersten Phase der Peritonealraum oder die Bauchhöhle des Patienten mit der Peritoneallösung befüllt. Die Steuereinheit 24 steuert die Sperrorgane hierzu derart an, daß das erste Sperrorgan 18 in der Verbindungsleitung 17 und das dritte Sperrorgan 22 in der Ablaufleitung 7 sowie das vierte Sperrorgan 23 in der Anschlußleitung 16 geöffnet sind. Das zweite Sperrorgan 21 in der Zulaufleitung 6 ist hingegen geschlossen. Zum Fördern der Peritoneallösung setzt die Steuereinheit 24 die okkludierende Pumpe 25 in Gang, die Peritoneallösung aus dem Beutel 14 durch die Anschlußleitung 16, die Zulaufleitung 6 in die erste Kammer 9a des Dialysators 10 fördert, aus der die Lösung dann sowohl über den ersten und zweiten Abschnitt 7a, 7b der Ablaufleitung 7 in das zweite Lumen 3 des Pertonealkatheters als auch über die Verbindungsleitung 17 und den ersten Abschnitt 6a der aus den Abschnitten 6a, 6b bestehen Zulaufleitung 6 in das erste Lumen 2 des Katheters 1 strömt. Der Peritonealraum wird somit über beide Lumen des Katheters befüllt.

Nach Ablauf eines ersten Zeitintervalls schließt die Steuereinheit 24 das erste und vierte Sperrorgan 18, 23 und öffnet das zweite Sperrorgan 21. Daraufhin pumpt die Pumpe 25 die Peritoneallösung über das erste Lumen 2 des Peritonealkatheters 1 und die Zulaufleitung 6 in die erste Kammer 9a des Dialysators 10, aus der die Lösung über die Ablaufleitung 7 und das zweite Lumen 3 des Katheters 1 wieder in den Peritonealraum abfließt. Die Pumpe 25 ist für ein zweites vorgegebenes Zeitintervall, das der Behandlungsdauer entspricht, in Betrieb.

Nach Ablauf der Behandlungsdauer öffnet die Steuereinheit 24 wieder das erste und vierte Sperrorgan 18, 23. Unter dem Einfluß der Schwerkraft strömt nun die Peritoneallösung aus dem Peritonealraum sowohl über das erste als auch das zweite Lumen 2, 3 des Peritonealkatheters in den Beutel 14. Die Lösung strömt einerseits über das erste Lumen 2, den ersten Abschnitt 6a der Zulaufleitung 6 und die Anschlußleitung 16 in den Beutel und andererseits über das zweite Lumen 3, den zweiten Abschnitt 7b der Ablaufleitung 7, die Bypassleitung 17 sowie den ersten Abschnitt 6a der Zulaufleitung in die Anschlußleitung. Dadurch wird auch der Auslauf der Flüssigkeit beschleunigt.

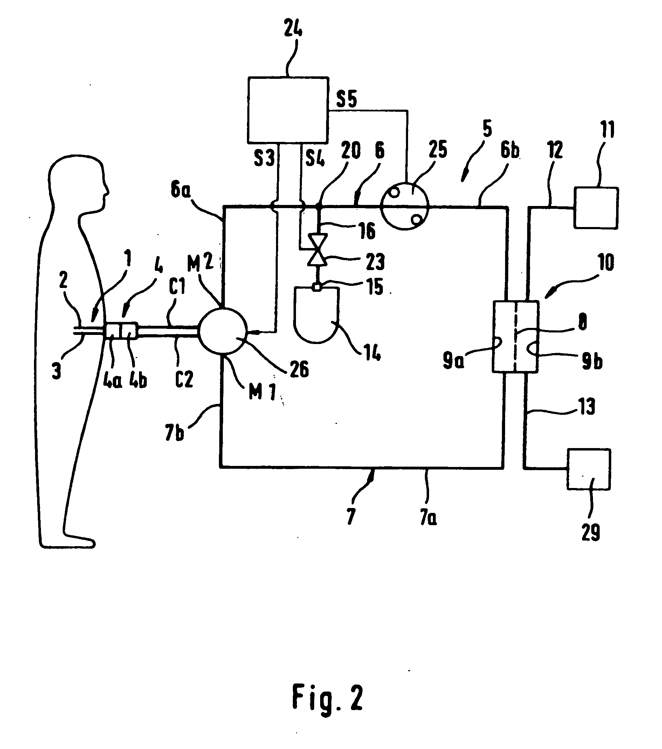

Figur 2 zeigt ein zweites Ausführungsbeispiel der Peritonealdialysevorrichtung. Diese Ausführungsform unterscheidet sich von dem unter Bezugnahme auf Figur 1 beschriebenen Ausführungsbeispiel dadurch, daß anstelle der Schlauchklemmen als Sperrorgan eine nach Art eines Schiebers ausgebildete Ventileinrichtung vorgesehen ist. Ansonsten ist der Aufbau identisch. Diejenigen Teile von Figur 2, die denjenigen von Figur 1 entsprechen, sind daher mit den gleichen Bezugszeichen versehen.

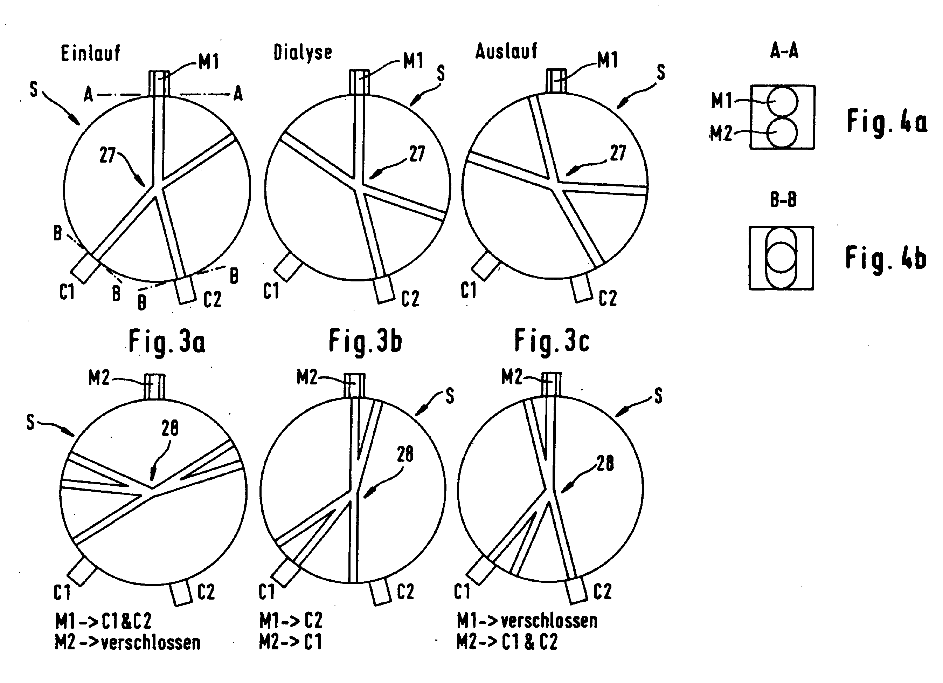

Die Ventileinrichtung 26 weist zwei Anschlüsse C1, C2 auf, an denen die zu dem ersten und zweiten Lumen 2, 3 des Peritonealkatheters 1 führenden Leitungsabschnitte der Zu- und Ablaufleitung 6, 7 angeschlossen sind. An den Anschlüssen Ml, M2 sind die zu dem Dialysator 10 führenden Abschnitte der Zu- und Ablaufleitung angeschlossen. Die Funktionsweise der Ventileinrichtung wird anhand der Figuren 3a bis 3c sowie 4a und 4b im einzelnen beschrieben.

Die Ventilanordnung weist eine in den Figuren nur andeutungsweise dargestellt Scheibe S auf, die um ihren Mittelpunkt drehbar gelagert ist und zur Einstellung der einzelnen Strömungsverbindungen vorzugsweise elektromagnetisch betätigt wird. Die Ansteuerung erfolgt mit der Steuereinheit 24. Die Scheibe S weist zwei übereinander liegende Kanalsysteme 27, 28 auf. Diese setzen sich jeweils aus mehreren radial verlaufenden Kanalabschnitten zusammen, die sich im Mittelpunkt der Scheibe treffen. Der besseren Übersichtlichkeit halber sind in den Figuren 3a bis 3c das in der oberen Ebene liegende Kanalsystem 27 und darunterliegende Kanalsystem 28 getrennt dargestellt.

Figur 4a zeigt einen Schnitt auf der Anschlußseite M1, M2. Der Anschluß M1 liegt auf der Höhe des oberen Kanalsystems 27, während der Anschluß M2 auf der Höhe des unteren Kanalsystems 28 liegt. Wenn einer der Kanalabschnitte des oberen bzw. unteren Kanalsystems 27, 28 auf den Anschluß M1 bzw. M2 ausgerichtet ist, kann Flüssigkeit in oder aus dem jeweiligen Kanalabschnitt strömen. Figur 4b zeigt einen Schnitt auf der Anschlußseite C1, C2. Eine Strömungsverbindung wird dann hergestellt, wenn entweder ein Kanalabschnitt des oberen als auch ein Kanalabschnitt des unteren Kanalsystems auf den jeweiligen Anschluß ausgerichtet ist. Beim Drehen der Scheibe ergeben sich die folgenden Ventilstellungen.

Figur 3a zeigt die erste Ventilstellung, in der der Anschluß M1 mit den Anschlüssen C1 und C2 verbunden ist. Anschluß M2 ist verschlossen. In dieser Ventilstellung erfolgt der Einlauf der Peritoneallösung in den Peritonealraum über die beiden Lumen des Peritonealkatheters.

Die Dialyse erfolgt in der Ventilstellung gemäß Figur 3b, in der Anschluß M1 mit Anschluß C2 und Anschluß M2 mit Anschluß C1 verbunden sind. Die Verbindungsleitung ist hierbei geschlossen.

Figur 3c zeigt die Ventilstellung für den Auslauf der Peritoneallösung. Anschluß M2 ist mit den Anschlüssen C1 und C2 verbunden. Anschluß M1 ist verschlossen. Die Peritoneallösung kann somit über beide Lumen des Katheters aus dem Peritonealraum strömen.

## Patentansprüche

1. Vorrichtung zur Peritonealdialyse zum Anschluß an einen implantierbaren Peritonealkatheter (1), der ein erstes Lumen (2) zum Zuführen bzw. Abführen einer Peritoneallösung und ein zweites Lumen (3) zum Abführen bzw. Zuführen der Peritoneallösung aus dem Peritonealraum aufweist, mit einem Überleitungsschlauchsystem (5), das eine mit dem ersten Lumen verbindbare Zulaufleitung (6) und eine mit dem zweiten Lumen des Peritonealkatheters verbundene Ablaufleitung (7) aufweist, und mit einer Einrichtung (14) zur Bereitstellung einer Peritoneallösung, **dadurch gekennzeichnet, dass** zum Einlauf oder Auslauf von Peritoneallösung in den bzw. aus dem Peritonealraum Mittel (17,18; 6,21; 7,22) zur Herstellung einer Strömungsverbindung zwischen der Zulaufleitung (6) oder der Ablaufleitung (7) und sowohl dem ersten Lumen (2) als auch dem zweiten Lumen (3) des Peritonealkatheters vorgesehen sind.

2. Vorrichtung zur Peritonealdialyse mit einem implantierbaren Peritonealkatheter (1), der ein erstes Lumen (2) zum Zuführen bzw. Abführen einer Peritoneallösung und ein zweites Lumen (3) zum Abführen bzw. Zuführen der Peritoneallösung aus dem Peritonealraum aufweist, mit einem Überleitungsschlauchsystem (5), das eine mit dem ersten Lumen verbundene Zulaufleitung (6) und eine mit dem zweiten Lumen des Peritonealkatheters verbundene Ablaufleitung (7) aufweist, und mit einer Einrichtung (15) zur Bereitstellung einer Peritoneallösung, **dadurch gekennzeichnet, dass** zum Einlauf oder Auslauf von Peritoneallösung in den bzw. aus dem Peritonealraum Mittel (17, 18; 6, 21; 7, 22) zur Herstellung einer Strömungsverbindung zwischen der Zulaufleitung oder der Ablaufleitung und sowohl dem ersten Lumen als auch dem zweiten Lumen des Peritonealkatheters vorgesehen sind.

3. Vorrichtung nach Anspruch 1 oder 2, **dadurch gekennzeichnet, dass** die Mittel zur Herstellung einer Strömungsverbindung zwischen der Zu- oder Ablaufleitung (6, 7) und dem ersten und zweiten Lumen (2, 3) eine Verbindungsleitung (17) zur Herstellung einer Strömungsverbindung zwischen dem ersten und zweiten Lumen (2, 3) mit einem Sperrorgan (18) zum Öffnen und Schließen derselben aufweisen.

4. Vorrichtung nach einem der Ansprüche 1 bis 3, **dadurch gekennzeichnet, dass** die Mittel zur Herstellung einer Strömungsverbindung zwischen der Zu- oder Ablaufleitung (6, 7) und dem ersten und zweiten Lumen (2, 3) ein zweites Sperrorgan (21) aufweisen, das in der Zulaufleitung (6) angeordnet ist.

5. Vorrichtung nach einem der Ansprüche 1 bis 4, **dadurch gekennzeichnet, dass** die Mittel zur Herstellung einer Strömungsverbindung zwischen der Zu- oder Ablaufleitung (6, 7) und dem ersten und zweiten Lumen (2, 3) ein drittes Sperrorgan (22) aufweisen, das in der Ablaufleitung (7) angeordnet ist.

6. Vorrichtung nach einem der Ansprüche 1 bis 5, **dadurch gekennzeichnet, dass** zwischen dem Peritonealkatheter (17) und dem Überleitungsschlauchsystem (5) ein Konnektor (4) angeordnet ist, so dass das Überleitungsschlauchsystem von dem Peritonealkatheter abnehmbar ist.

7. Vorrichtung nach einem der Ansprüche 1 bis 6, **dadurch gekennzeichnet, dass** die Zulaufleitung (6) mit einem Einlass einer ersten Kammer (9a) eines durch einen Membran (8) in die erste Kammer (9a) und eine zweite Kammer (9b) unterteilten Dialysators (10) verbunden ist, und die Ablaufleitung (7) mit einem Auslass der ersten Kammer (9a) verbunden ist, und dass ein Einlass der zweiten Kammer (9b) mit einer Einrichtung (11) zur Bereitstellung von Dialysierflüssigkeit und ein Auslass der zweiten Kammer mit einem Ablauf (29) verbunden ist.

8. Vorrichtung nach einem der Ansprüche 1 bis 7, **dadurch gekennzeichnet, dass** in die Ablaufleitung (7) eine Pumpe (25) insbesondere eine okkludierende Pumpe geschaltet ist.

9. Vorrichtung nach einem der Ansprüche 1 bis 8, **dadurch gekennzeichnet, dass** die Einrichtung zur Bereitstellung der Peritoneallösung ein Behältnis (14) mit einem Ein-/Auslass (15) ist, das über eine Anschlussleitung (16) mit der Zulaufleitung (6) verbunden ist.

10. Vorrichtung nach Anspruch 9, **dadurch gekennzeichnet, dass** in der Anschlussleitung (16) ein viertes Sperrorgan (23) vorgesehen ist.

11. Vorrichtung nach Anspruch 10, **dadurch gekennzeichnet, dass** die Anschlussleitung (16) mit der Zulaufleitung (6) an einem Verbindungspunkt (20) zwischen Peritonealkatheter (1) und Pumpe (25) verbunden ist.

12. Vorrichtung nach einem der Ansprüche 1 bis 11, **dadurch gekennzeichnet, dass** die Sperrorgane (18, 21, 22, 23) elektromagnetisch oder pneumatisch betätigbare Schlauchklemmen sind.

13. Vorrichtung nach Anspruch 12, **dadurch gekennzeichnet, dass** eine die Sperrorgane (18, 21, 22, 23) ansteuernde Steuereinheit (24) vorgesehen ist, die derart ausgebildet ist, dass
in einem ersten Zeitintervall zum Einlauf der Peritoneallösung in den Peritonealraum das zweite Sperrorgan (21) geschlossen und das erste, dritte und vierte Sperrorgan (18, 21, 22, 23) geöffnet sind,
in einem zweiten Zeitintervall zur Durchführung der Dialyse das erste und vierte Sperrorgan (18, 23) geschlossen und das zweite und dritte Sperrorgan (21, 22) geöffnet sind, und
in einem dritten Zeitintervall zum Auslauf der Peritoneallösung aus dem Peritonealraum das erste, zweite und vierte Sperrorgan (18, 21, 23) geöffnet sind.

14. Vorrichtung nach einem der Ansprüche 1 bis 13, **dadurch gekennzeichnet, dass** die Sperrorgane eine nach Art eines Drehschiebers ausgebildete Ventilanordnung (26) sind.

## Claims

1. Apparatus for peritoneal dialysis, for connection to an implantable peritoneal catheter (1) which has a first lumen (2) for the infeed or outfeed of a peritoneal solution from the peritoneal cavity and a second lumen (3) for the infeed or outfeed of the peritoneal solution therefrom, having a system (5) of transfer tubing which has an input line (6) able to be connected to the first lumen of the peritoneal catheter and an output line (7) connected to the second lumen thereof, and having an arrangement (14) for supplying a peritoneal solution, **characterised in that**, for the inlet or outlet of peritoneal solution to or from the peritoneal cavity, means (17, 18; 6, 21; 7, 22) are provided for making a connection for flow between the input line (6) or the output line (7) and both the first lumen (2) and the second lumen (3) of the peritoneal catheter.

2. Apparatus for peritoneal dialysis having an implantable peritoneal catheter (1) which has a first lumen (2) for the infeed or outfeed of a peritoneal solution from the peritoneal cavity and a second lumen (3) for the infeed or outfeed of the peritoneal solution therefrom, having a system (5) of transfer tubing which has an input line (6) connected to the first lumen of the peritoneal catheter and an output line (7) connected to the second lumen thereof, and having an arrangement (15) for supplying a peritoneal solution, **characterised in that**, for the inlet or outlet of peritoneal solution to or from the peritoneal cavity, means (17, 18; 6, 21; 7, 22) are provided for making a connection for flow between the input line or the output line and both the first lumen and the second lumen of the peritoneal catheter.

3. Apparatus according to claim 1 or 2, **characterised in that** the means for making a connection for flow between the input or output line (6, 7) and the first and second lumens (2, 3) have a connecting line (17) for making a connection for flow between the first and second lumens (2, 3) which has a shut-off member (18) for opening and closing the said line.

4. Apparatus according to one of claims 1 to 3, **characterised in that** the means for making a connection for flow between the input or output line (6, 7) and the first and second lumens (2, 3) have a second shut-off member (21) which is arranged in the input line (6).

5. Apparatus according to one of claims 1 to 4, **characterised in that** the means for making a connection for flow between the input or output line (6, 7) and the first and second lumens (2, 3) have a third shut-off member (22) which is arranged in the output line (7).

6. Apparatus according to one of claims 1 to 5, **characterised in that** a connector (4) is arranged between the peritoneal catheter (17) and the system of transfer tubing (5), thus allowing the system of transfer tubing to be disconnected from the peritoneal catheter.

7. Apparatus according to one of claims 1 to 6, **characterised in that** the input line (6) is connected to an inlet of a first chamber (9a) of a dialyser (10), which dialyser (10) is divided by a membrane (8) into the first chamber (9a) and a second chamber (9b), and the output line (7) is connected to an outlet of the first chamber (9a), and **in that** an inlet of the second chamber (9b) is connected to an arrangement (11) for supplying dialysis fluid and an outlet of the second chamber is connected to a drain outlet (29).

8. Apparatus according to one of claims 1 to 7, **characterised in that** a pump (25), and in particular an occluding pump, is connected into the output line (7).

9. Apparatus according to one of claims 1 to 8, **characterised in that** the arrangement for supplying the peritoneal solution is a container (14), having an inlet/outlet (15), which is connected to the input line (6) via a connecting line (16).

10. Apparatus according to claim 9, **characterised in that** a fourth shut-off member (23) is provided in the connecting line (16).

11. Apparatus according to claim 10, **characterised in that** the connecting line (16) is connected to the input line (6) at a connecting point (20) between the peritoneal catheter (1) and the pump (25).

12. Apparatus according to one of claims 1 to 11, **characterised in that** the shut-off members (18, 21, 22, 23) are tube clamps which are able to be actuated electromagnetically or pneumatically.

13. Apparatus according to claim 12, **characterised in that** a control unit (24) is provided which operates the shut-off members (18, 21, 22, 23) and which is so designed that
in a first interval of time, the second shut-off member (21) is closed and the first, third and fourth shut-off members (18, 21, 22, 23) are open for the inlet of the peritoneal solution to the peritoneal cavity,
in a second interval of time, the first and fourth shut-off members (18, 23) are closed and the second and third shut-off members (21, 22) are open to allow the dialysis to be carried out, and
in a third interval of time, the first, second and fourth shut-off members (18, 21, 23) are open for the outlet of the peritoneal solution from the peritoneal cavity.

14. Apparatus according to one of claims 1 to 13, **characterised in that** the shut-off members are a valve arrangement (26) designed after the fashion of a rotary disc valve.

## Revendications

1. Appareil de dialyse péritonéale pour un raccordement à un cathéter péritonéal (1) pouvant être implanté qui comprend une première lumière (2) pour alimenter et/ou évacuer une solution péritonéale et une deuxième lumière (3) pour évacuer et/ou alimenter de la solution péritonéale à partir de la cavité péritonéale, et avec un système de tuyau de transmission (5) qui comprend une conduite d'arrivée (6) pouvant être raccordée à la première lumière et une conduite de décharge (7) reliée à la deuxième lumière du cathéter péritonéal, avec un dispositif (14) de mise à disposition d'une solution péritonéale, **caractérisé en ce que** des moyens (17,18 ; 6,21 ; 7,22) sont prévus pour l'admission ou la sortie d'une solution péritonéale dans et/ou hors de la cavité péritonéale pour fabriquer une liaison fluidique entre la conduite d'arrivée (6) ou la conduite de décharge (7) et aussi bien la première lumière (2) que la deuxième lumière (3) du cathéter péritonéal.

2. Appareil de dialyse péritonéale avec un cathéter péritonéal (1) pouvant être implanté qui comprend une première lumière (2) pour alimenter et/ou évacuer une solution péritonéale et une deuxième lumière (3) pour évacuer et/ou alimenter de la solution péritonéale à partir de la cavité péritonéale, et avec un système de tuyau de transmission (5) qui comprend une conduite d'arrivée (6) raccordée à la première lumière et une conduite de décharge (7) reliée à la deuxième lumière du cathéter péritonéal, avec un dispositif (14) de mise à disposition d'une solution péritonéale, **caractérisé en ce que** des moyens (17,18 ; 6,21 ; 7,22) sont prévus pour l'admission ou la sortie d'une solution péritonéale dans et/ou hors de la cavité péritonéale pour fabriquer une liaison fluidique entre la conduite d'arrivée (6) ou la conduite de décharge (7) et aussi bien la première lumière (2) que la deuxième lumière (3) du cathéter péritonéal.

3. Appareil selon la revendication 1 ou 2, **caractérisé en ce que** les moyens de fabrication d'une liaison fluidique entre la conduite d'arrivée ou de décharge (6, 7) et la première et deuxième lumière (2, 3) comprennent une conduite de raccordement (17) servant à fabriquer une liaison fluidique ente la première et la deuxième lumière (2, 3) avec un élément de blocage (18) pour ouvrir et fermer celles-ci.

4. Appareil selon l'une des revendications 1 à 3, **caractérisé en ce que** les moyens de fabrication d'une liaison fluidique entre la conduite d'arrivée ou de décharge (6, 7) et la première et deuxième lumière (2, 3) comprennent un deuxième élément de blocage (21) qui est disposé dans la conduite d'arrivée (6).

5. Appareil selon l'une des revendications 1 à 4, **caractérisé en ce que** les moyens de fabrication d'une liaison fluidique entre la conduite d'arrivée ou de décharge (6, 7) et la première et deuxième lumière (2, 3) comprennent un troisième élément de blocage (21) qui est disposé dans la conduite de décharge (7).

6. Appareil selon l'une des revendications 1 à 5, **caractérisé en ce qu'**un connecteur (4) est disposé entre le cathéter péritonéal (17) et le système de tuyau de transmission (5) de sorte que le système de tuyau de transmission (5) du détaché du cathéter péritonéal.

7. Appareil selon l'une des revendications 1 à 6, **caractérisé en ce que** la conduite d'arrivée (6) est reliée à un orifice d'admission d'une première chambre (9a) d'un dialyseur (10) divisé par une membrane (8) en une première chambre (9a) et une deuxième chambre (9b), et la conduite de décharge (7) est reliée à un orifice de sortie de la première chambre (9a), et **en ce qu'**un orifice d'entrée de la deuxième chambre (9b) est relié à un dispositif (11) de mise à disposition d'un liquide de dialyse et un orifice de sortie de la deuxième chambre est relié à une évacuation (29).

8. Appareil selon l'une des revendications 1 à 7, **caractérisé en ce qu'**une pompe (25), en particulier une pompe formant occlusion, est enclenchée dans la conduite de décharge (7).

9. Appareil selon l'une des revendications 1 à 8, **caractérisé en ce que** le dispositif de mise à disposition de la solution péritonéale est un récipient (14) avec une entrée/sortie (15) qui est reliée via une conduite de raccordement (16) à la conduite d'arrivée (6).

10. Appareil selon la revendication 9, **caractérisé en ce qu'**on prévoit un quatrième élément de blocage (23) dans la conduite de raccordement (16).

11. Appareil selon la revendication 10, **caractérisé en ce que** la conduite de raccordement (16) est reliée à la conduite d'arrivée (6) au niveau d'un point de liaison (20) entre le cathéter péritonéal (1) et la pompe (25).

12. Appareil selon l'une des revendications 1 à 11, **caractérisé en ce que** les éléments de blocage (18, 21, 22, 23) sont des pinces pour tuyaux souples pouvant être actionnées de façon électromagnétique ou pneumatique.

13. Appareil selon la revendication 12, **caractérisé en ce qu'**une unité de commande (24) contrôlant l'élément de blocage (18, 21, 22, 23) est prévue qui est conçue de telle manière que
◆ le deuxième élément de blocage (21) est fermé dans un premier intervalle de temps pour introduire la solution péritonéale dans la cavité péritonéale et les premier, troisième et quatrième éléments de blocage (18, 21, 22, 23) sont ouverts,
◆ les premier et quatrième éléments de blocage (18, 23) sont fermés dans un deuxième intervalle de temps pour exécuter la dialyse et les deuxième et troisième éléments de blocage (21, 22) sont ouverts, et
◆ les premier, deuxième et quatrième éléments de blocage (18, 21, 23) sont ouverts dans un troisième intervalle de temps pour évacuer la solution péritonéale hors de la cavité péritonéale.

14. Appareil selon l'une des revendications 1 à 13, **caractérisé en ce que** les éléments de blocage sont un ensemble de valves (26) conçu selon un type de tiroir rotatif.
